# EUROPEAN PATENT SPECIFICATION

(11) **EP 1 013 239 B1**
(45) Date of publication and mention of the grant of the patent: **16.02.2005**
(21) Application number: 99310315.9
(22) Date of filing: 21.12.1999
(51) Int. Cl.: A61F 2/08

(54) **Graft material convenience package**
Ligamenttransplantate und Verpackung
Prothèse ligamentaire et son emballage

(30) Priority: 22.12.1998 US 113312 P
(43) Date of publication of application: 28.06.2000
(73) Proprietor: DEPUY ORTHOPAEDICS, INC., Warsaw, Indiana 46581-0988 (US)
(72) Inventor: Enzerink, Robert-Jan, Davis, CA 95616 (US); Wehrly, Peter, Brentwood, CA 94513 (US)
(74) Representative: Belcher, Simon James

(56) References cited:
- WO-A-90/02532
- FR-A- 2 662 600
- US-A- 5 031 762
- US-A- 5 067 962
- US-A- 5 397 357
- US-A- 5 562 669
- US-A- 5 607 476
- US-A- 5 733 289
- US-A- 5 782 914

## Description

The present invention relates to a convenient packaged ligament graft; more particularly to a sterilized preserved ready to use ligament graft; and most particularly to a convenient sterilized preserved ready to use ligament graft for replacement of a cruciate ligament. The present invention also relates to methods of preparing and using the same.

In replacing an anterior or posterior cruciate ligament, standard techniques often involve drilling bone tunnels through the tibia and femur, inserting replacement ligament material in the bone tunnels, and securing the ends. An example of such a technique is disclosed in US-5354300. Known techniques include replacement if ligaments that have bone plugs at one or both ends. Replacement ligaments including bone plugs are particularly useful, as the bone plugs fuse into the prepared bone tunnel, healing quickly and providing a secure attachment for the replacement ligament. Replacement ligaments may be anchored in the bone tunnels by interference screw, cross-pin, tab-loop anchor, screw-and-washer, or a variety of other means as disclosed in, for example, US-5562671 and US-4950270.

Prior art replacement ligaments often involve autografts, for which suitable material is harvested from elsewhere in the patient's body. The patella tendon is widely used for such graft material, as it can be harvested with bone plugs at both ends. However, patients often experience considerable pain at the donor site following harvest of this tendon.

Furthermore, an autograft patella tendon is not always available for use, particularly in revision surgery. Autograft alternatives to the patella tendon include the semitendinosis and gracilis hamstring tendons, the central quadriceps tendon, and fascia lata. See, for example, Charles H. Brown Jr. and Joseph H. Sklar, "Graft Selection, Nonpatellar Alternatives Gain Popularity," BioMechanics, June 1998, at 21; John P. Fulkerson and Rolf Langeland, "An Alternative Cruciate Reconstruction Graft: The Central Quadriceps Tendon," Arthroscopy: The Journal of Arthroscopic and Related Surgery, June 1995, at 252. However, these replacement ligaments cannot be harvested with bone plugs at both ends. Thus, when using a replacement ligament of this type, the graft material is often sutured to a bone plug prior to insertion in the bone tunnel. Assembly of the replacement ligament during surgery uses significant operating room time and contributes considerably to the expense of such surgery.

Allografts may be used as replacement ligaments. However, the supply of allograft patella tendons is limited. Even when patella tendons are available, additional preparation is often required prior to use. As with autografts, available allograft semitendinosis, gracilis, quadriceps, Achilles' tendons, and flexor and extensor tendons (usually from the foot) require considerable additional preparation prior to use.

US-5565669 discusses a method of ligament reconstruction. A ligament replacement extends between two bone plugs which are fixed within bone tunnels in the bones using interference screws. The ligament may be a semitendinous tendon attached to itself to form a loop attached to each of the two bone plugs.

US-5031762 relates to a three envelope package for sterile tissue specimens or other sterile objects. The sterile tissue is sealed inside the sterile interior of an innermost envelope which is itself sealed within the sterile interior of an intermediate envelope equipped with a peel back seal. The intermediate envelope is then sealed inside the sterile interior of an outermost envelope which is impermeable to a storage medium.

US-5067929 discusses a bioprosthetic ligament. The ligament is harvested with a bone piece at at least one end to preserve intact a substantial portion of the natural ligament-to-bone attachment structure.

US-5397357 relates to a method of preparing a bone-tendon-bone core graft. A harvested bone core is divided into two halves which are inserted onto holding pins movably spaced on work station. A tendon is then secured with suture onto the bone core halves to form the bone-tendon-bone core graft.

US-5607476 discusses the processing of fibrous connective tissue. The tissue is made substantially antigen-free by contact with one or more extraction agents.

According to the present invention, graft material is packaged for convenient use. Packaged, sterilized, ready-to-use replacement grafts are provided in a variety of lengths with pre-attached sutures for easy insertion. Thus, a surgeon may select a graft of the appropriate configuration, length, and size for use during reconstructive surgery, thereby eliminating the surgery time previously spent on harvesting and/or constructing the graft.

Long strands of sutures may be attached to one or both ends, in order to facilitate insertion. The replacement ligament material may include semitendinosis, gracilis, or quadriceps tendon, or other allograft or xenograft material. Other organic materials, such as small intestine submucosa ("SIS"), collagen scaffolds, or synthetic materials may be used, as disclosed in US-4902508 and US-5711969. A packaged ligament graft according to the preamble of claim 1 has been disclosed in FR 2 662 600.

According to a first aspect of the invention, as defined in appended claim 1, replacement ligament is provided without bone plugs. In this aspect, the graft material may be cut to appropriate lengths and widths, looped, and sutured. This aspect may be particularly appropriate for use with fixation techniques which do not use bone plugs, for instance in surgical situations wherein a crosspin is used to capture a looped replacement ligament.

According to a second aspect of the present invention, there is provided a method of preparing a replacement graft package as defined in appended claim 7, including the steps of cutting and shaping a harvested piece of graft material, then assembling, preserving, and packaging the replacement graft.

The bone and tissue for use in this invention may be obtained from tissue banks, such as LifeNet, Virginia Beach, Va. The product may be preserved by freeze drying prior to packaging and reconstituted at the time of surgery. Water or a saline solution may be used for the reconstitution. Alternatively, the replacement ligament may be frozen without drying and thawed just prior to use.

In another embodiment of the invention, the replacement ligament is used to repair a damaged cruciate ligament. A sterile graft of appropriate size is selected and inserted in pre-drilled femoral and tibial holes. After insertion, the graft can be fixed within the knee using fixation devices including interference screws, cross-pins, tab-loop anchors, and screws and washers.

According to a third aspect of the invention, defined in appended claim 17, there is provided a kit for repairing a damaged ligament comprising the pre-packaged ligament according to the first aspect of this invention, pre-attached sutures, and a graft fixation devices including screws, cross-pins, tab-loop anchors, screws and washers, or other fixation devices.

While this invention is described for use in reconstruction of the cruciate ligaments of the knee, it should be appreciated that the invention may be practiced in other manners as well, including in the replacement of other soft tissue, especially in the replacement of other ligaments.

Embodiments of the present invention will now be described by way of example with reference to the accompanying drawings, in which
Fig. 1 is a perspective view of a replacement ligament which does not form part of the claimed invention but serves as an example to help understand the present invention, wherein a single piece of graft material is looped three times around a pair of bone plugs;
Fig. 2 is similar to Fig. 1, except showing a line drawing representing the graft material and illustrating the direction of looping;
Fig. 3 is a sectional view along the line of 3-3 of Fig. 1;
Fig. 4 is similar to Fig. 1, except the graft material comprises a single strand which is looped around each bone plug twice;
Fig. 5 is a perspective view of an end of the replacement ligament of Fig. 4 showing the sutures extending through the construct;
Fig. 6 is a sectional view along the line of 6-6 of Fig. 5;
Fig. 7 is similar to Fig. 6, except showing an alternate graft material arrangement;
Fig. 8 is an embodiment of the replacement ligament for use in this invention, showing a replacement ligament without bone plugs;
Fig. 9 is a sectional view along the line of 9-9 of Fig. 8;
Fig. 10 is similar to Fig. 8, except the replacement ligament includes a looped end;
Fig. 11 is given as an example to help understanding of the present invention, and is similar to Fig. 8, except the replacement ligament includes one bone plug;
Fig. 12 is a sectional view across 12-12 of Fig. 11.
Fig. 13 is similar to Fig. 12, except showing an alternate graft material arrangement;
Fig. 14 is similar to Fig. 11, except the replacement ligament includes bone plugs at both ends; and
Fig. 15 shows the graft of Fig. 8 as it is being inserted and fixed into the knee of a patient.

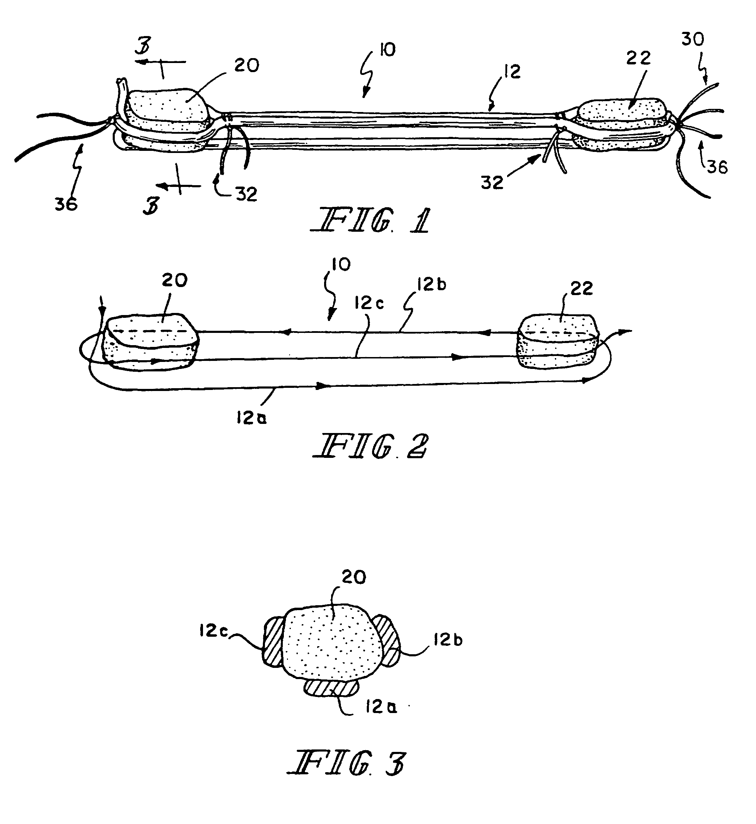

Referring to the drawings, Fig. 1 shows generally a replacement ligament 10 which does not form part of the claimed invention but serves as example to help understand the present invention. In this example, replacement ligament 10 comprises graft material 12 and a set of bone plugs 20 and 22. Replacement ligament 10 of this example also includes a set of interior sutures 32 for securing bone plugs 20 and 22 in place, and a set of end sutures 36 for use in insertion of replacement ligament 10 into a patient. Graft material 12 provides suitable flexibility and strength for ligament replacement, and bone plugs 20 and 22 are provided for bone ingrowth.

Still referring to Fig. 1, end sutures 36 can be used to secure the ends of graft material 12 in place, while interior sutures 32 secure portions of graft material 12 together to create pockets for receiving the bone plugs 20 and 22. Additionally, a long strand suture 30 can be secured to one or both ends of the replacement ligament. Such additional long strand sutures 30 can be used later to aid in insertion of replacement ligament 10 into prepared bone tunnels in the knee of the patient.

Fig. 2 illustrates a method for constructing replacement ligament 10 of Fig. 1. Graft material 12 is wrapped around first bone plug 20 to second bone plug 22, as shown by a line representing graft material 12a. Graft material 12b then loops back around second bone plug 22 and to first bone plug 20. Finally, graft material 12c wraps around first bone plug 20 and back past second bone plug 22. Thus, in this illustrative embodiment the graft material 12 is triple-stranded.

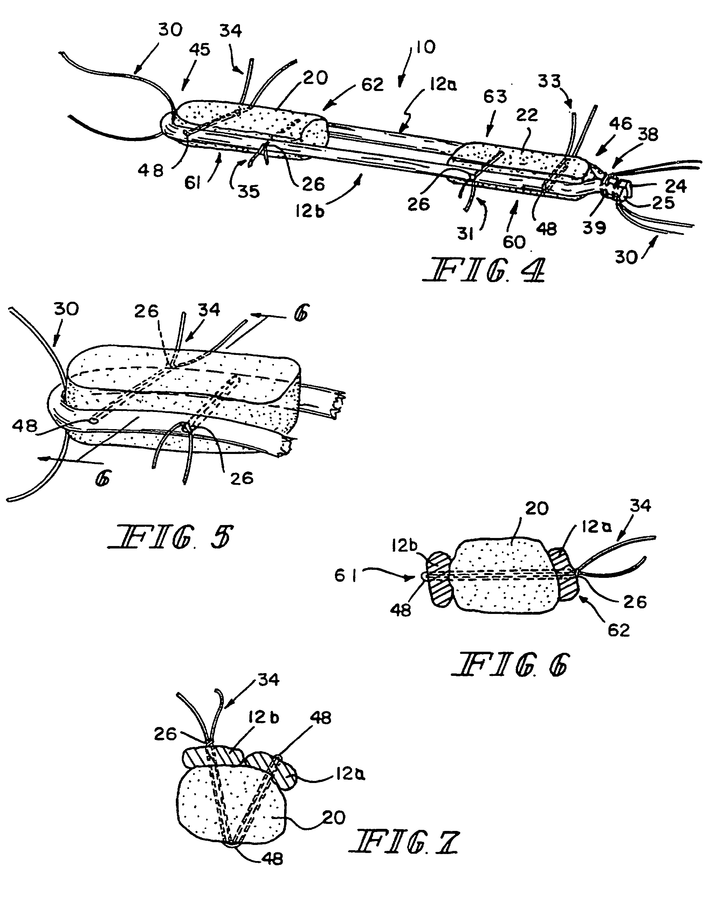

Fig. 3 shows a cross section of Fig. 1 illustrating a possible placement of graft material 12a, 12b, and 12c around bone plug 20. As illustrated in Figs. 1 and 3, bone plugs 20 and 22 are exposed on only approximately one third of the circumference. However, in other embodiments, more surface area of the bone plugs may be exposed. This may be accomplished by looping the replacement ligament 12 twice rather than three times, as illustrated in Figs. 4-7.

An alternative example that provides additional bone surface area is shown in Fig. 4. In this example which is also not a part of the claimed invention, replacement ligament 10 is provided with two bone plugs 20 and 22. Graft material 12 is double stranded. The first strand, graft material 12a, is located along a side 60 of bone plug 22, extends to bone plug 20, and is located along a side 61 of bone plug 20. Graft material 12 then loops around a proximal end 45 of bone plug 20, and the second strand, graft material 12b, is located along a second side 62 of bone plug 20, and then extends to bone plug 22 at a side 63. As illustrated, bone plug 20 is then secured to graft material 12 by a set of sutures 34 and 35. Suture 34 extends from side 62 of bone plug 20 and passes completely through bone plug 20 to side 61 where optionally a stitch 48 is made. Suture 34 then returns to side 62. As shown, suture 35, is provided in the opposite orientation and extends from side 61 of bone plug 20 and passes completely through to side 62. Suture 35 then passes back through bone plug 20 to side 61 where a knot 26 is tied. As described above, graft material 12 is also wrapped around bone plug 22. Bone plug 22 is then secured to replacement ligament 12 by sutures 31 and 33 that pass completely through bone plug 22 in the same manner as sutures 34 and 35. A set of bundle sutures 38, preferably provided in a whip stitch 39, may be used to secure ends 24 and 25 of graft material 12 beyond bone plug 22 at a distal end 46 of replacement ligament 10. Additional long strand sutures 30 may be secured to the proximal and distal ends 45 and 46 of the replacement ligament 10. It will be understood that the terms proximal and distal are used for convenience and represent the preferred orientation of replacement ligament 10 for use in anterior cruciate ligament repair. Alternative graft orientations may be required for particular applications or surgeon preference.

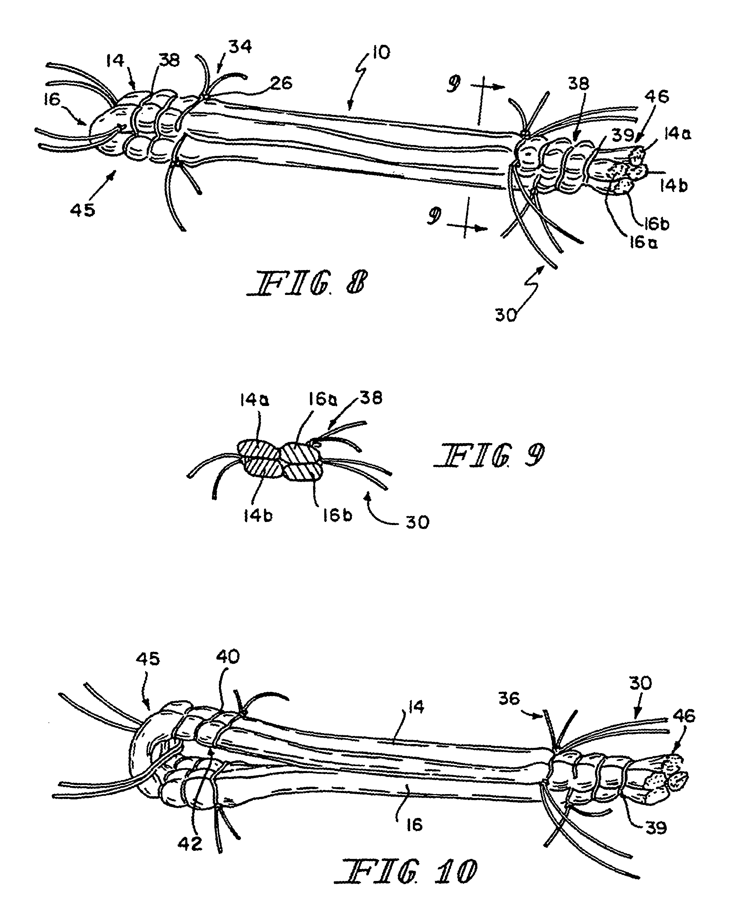

Figs. 5 and 6 further illustrate a construction in which graft material 12 sutured to bone plug 20 of Fig. 4. As best seen in Fig. 5, suture 34 passes completely through bone plug 20 twice, first passing from side 62 through bone plug 20 to side 61. Preferably a stitch 48 is then made on side 61. The suture then passes back through bone plug 20 to side 62. The ends of suture 34 are then tied in a knot 26, thus securing the opposing strands 12a and 12b of graft material 12. In the embodiments of Figs. 4-6, it is preferred that all strands of graft material 12 be secured to bone plug 20. However, for some applications surgeons may prefer replacement ligaments for which the bone plugs are provided in pockets, rather than rigidly sutured to the graft material.

In some fixation methods, it would be preferable to have bone exposed on both sides of the graft, as shown in Figs. 4-6. With other fixation methods, it may be preferable to have the greatest possible exposed surface area of bone plug 20 on one side, as seen in Fig. 7. Fig. 7 shows an alternative graft arrangement in which the graft strands 12a and 12b are placed together on one side of bone plug 20 and secured by suture 34. Also, the replacement ligament may be provided in a straight single length and sutured to bone plugs without looping at all. Finally, by varying the size of the bone plugs in relation to the replacement ligament width, more of the circumference of the bone plug may be exposed without sacrificing necessary strength of the replacement ligament.

An embodiment of the present invention is illustrated in Fig. 8. In this embodiment, a replacement ligament 10 is provided without bone plugs. Graft material 12 may be single-stranded or multiple-stranded. If graft material 12 is multiple-stranded as shown, the multiple strands may be created by bundling individual strands, by folding a strand back upon itself, or by a combination of both. Fig. 9 shows a cross section of Fig. 8 illustrating the multiple-stranded bundle created by the individual strands 14 and 16 folded back upon themselves and secured by bundle sutures 38.

Still referring to Fig. 8, multiple stranded replacement ligament graft distal end 46 maybe secured using bundle sutures 38, preferably provided in a whip stitch 39. As shown, 14a, 14b, 16a, and 16b are secured with bundle sutures 38 in a whip stitch 39 at both proximal and 45 and distal end 46. Also as shown, long strand sutures 30 are provided as separate sutures. Alternatively, long strand sutures 30 may be provided as the ends of bundle sutures 38. For some applications, long strand sutures 30 may be omitted. In the illustrative embodiment of Figs. 8 and 9, the replacement ligament 10 is made of two individual strands 14 and 16, which are folded back upon themselves at proximal end 45, creating a quadruple-strand graft. However, it will be understood that other strand arrangements are within the scope of this invention.

Fig. 10 shows an embodiment similar to that illustrated in Fig. 8. However, at proximal end 45, a set of semi-bundle sutures 40 only partially bundle graft material 12, creating a loop 42. Loop 42 may be particularly useful for fixation methods which employ a cross pin. Alternatively, loop 42 may be provided in applications where autograft bone plugs are preferred. A similar loop may be provided at distal end 46.

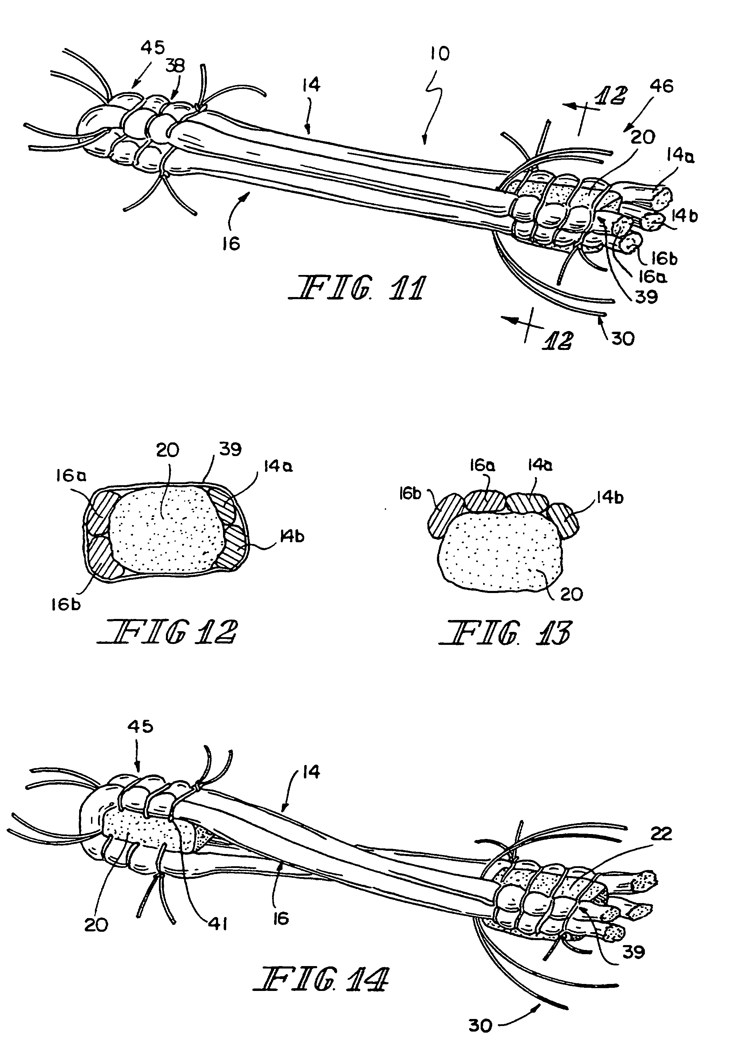

Fig. 11 illustrates an example, which again is not part of the claimed invention but useful to understand the invention which was prepared in a manner similar to the embodiment illustrated in Fig. 8, but includes one bone plug 20. In this illustrative example, bone plug 20 is included within the whip stitching 39 of bundle sutures 38 at distal end 46 of replacement ligament 10. The whip stitching 39 of bundle sutures 38 may provide enough support to hold the bone plug 20 in place. Alternatively, bone plug sutures similar to those shown in Fig. 5 may be necessary to provide proper attachment strength. Other arrangements for bone plug 20 are possible within the scope of this invention. Also, the proximal end 45 of this example may be looped as in Fig. 10, or closed as in Fig. 8.

A cross section of Fig. 11, shown in Fig. 12, illustrates a possible placement of replacement ligaments 14 and 16 around bone plug 20. The whip stitching 39 of the bundle sutures 38 secures bone plug 20 within the replacement ligament strands 14a, 14b, 16a, and 16b. Fig. 13 shows a cross section of an alternative arrangement to Fig. 12 wherein all four strands of ligaments 14 and 16 may be located on one side of bone plug 20. The stitching of the bundle sutures is not shown. Alternatively, ligaments 14 and 16 may be secured with sutures similar to those shown in Fig. 7.

As illustrated in Figs. 11-13, the graft material is provided in the quadruple strand arrangement of Fig. 8. However, other multiple strand arrangements may be suitable.

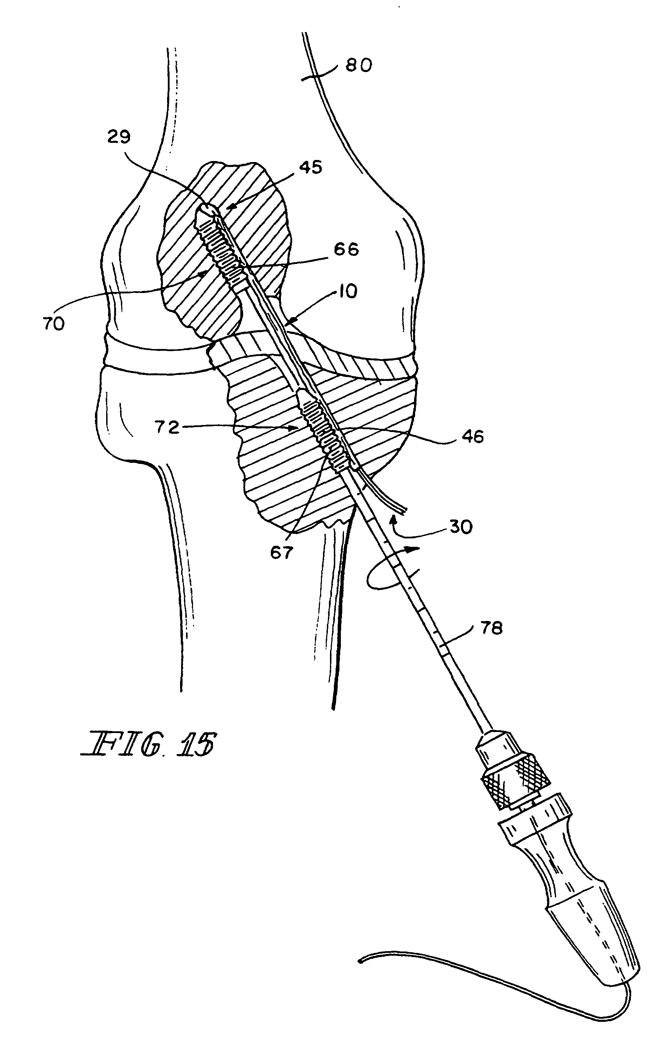

Fig. 14 illustrates another example not forming part of the claimed invention which is similar to that illustrated in Fig. 11, as the replacement ligaments 14 and 16 are looped at proximal end 45 and bone plug 22 is incorporated within the whip stitching 39 of bundle sutures 38. However, with the example illustrated in Fig. 14, bone plugs 20 and 22 are provided on both ends. In this embodiment, the replacement ligaments 14 and 16 of proximal end 45 are held together with a set of bundle sutures 41. As shown, the same sutures 41 are used to suture bone plug 20. This example ensures that bone plug 22 will not move during insertion into a patient's knee. Other methods of securing bone plug 20 are possible.

According to another example not forming part of the claimed invention, allograft replacement ligaments may be prepared using patella tendons which are harvested with bone plugs (not shown). Preparation of these replacement ligaments includes pre-attached sutures to facilitate subsequent insertion. The sutures may be made of absorbable or non-absorbable suture material. These replacement ligaments are provided in a variety of sizes and are packaged and sterilized for convenient use. The package may also include graft fixation devices.

In a method of construction of this invention, graft material may be obtained from a variety of sources including allograft, xenograft, or synthetic material. If allograft or xenograft material is used, it can be prepared by removing extraneous tissue, cutting the material to the proper size, and assembling into ready to use configurations, as described above.

Prior to assembly, the replacement ligament material may go through a series of washes. Such washes may include mixed detergent solutions (such as those sold under the trade mark ALLOWASH), sterile water, isopropyl alcohol, antibiotic solution, and a final rinse with sterile water. Mixed detergent solutions including those sold under the trade mark ALLOWASH are disclosed in US-5820581 and can include detergents such as (1) polyoxyethylene-4-lauryl ether having the chemical formula C₉H₁₉(OCH₂CH₂)₄OH, (2) octylphenol-ethyleneoxide, and (3) poly(ethylene glycol)p-nonyl-phenol-ether. The '581 patent also discloses various alternative cleaning solutions and washing protocols which may be used in the practice of this invention. The antibiotic solution may be a solution of endotoxin-free deionized/distilled water or ethanol, containing antibiotics, antiviral agents, hydrogen peroxide, permeation enhancers, organic acids, or a dilute solution of strong acids. Preferably, the antibiotic solution contains a mixture of bacitracin and polymyxin in sterile water. U.S. Patent No. 5,797,871, describes other methods of cleaning allograft bone. It should be understood that these and other techniques for cleaning the allograft tissue are within the scope of this invention.

Following assembly, the graft material may be placed under tension. For example, a tension spring may be used. This tension keeps the replacement ligament in proper alignment during subsequent preservation and packaging. The spring or other tension device may be removed before packaging or just prior to use.

Once the allograft or xenograft replacement ligaments have been washed, they are preserved. In one preferred embodiment of preparing the graft material, preservation is accomplished by a lyophilization cycle which may, for instance, last five days. Preferably, the replacement ligament is placed in sterile gauze within a sterile bottle prior to lyophilization, and the bottle is stoppered under vacuum following lyophilization to finish the packaging. When used, the replacement ligament may be reconstituted in either sterile water or saline, and such reconstitution may take approximately one hour under vacuum. For grafts which have been bottled under vacuum, this reconstitution may take place simply by introducing water or saline into the bottle by use of a sterile needle. Preferably, the needle would pierce the stopper without breaking the vacuum.

Alternatively, following washing, the replacement ligaments may be preserved by being fresh frozen. In this method of preservation, the replacement ligaments preferably are placed in a peelable soft package. Air is removed from the package by suction, and the package can be heat-sealed. A second, slightly larger, soft package may be used for additional protection of the graft material. For further sterilization, the replacement ligaments may be irradiated as is known in the art, for example with gamma radiation. Finally, the replacement ligaments are stored frozen, for instance from about -70°C to about -80°C, and may be distributed on dry ice. When needed, frozen grafts are thawed, preferably in sterile saline. Because fresh frozen replacement ligaments are not fully dried, the reconstitution step required for lyophilized grafts may be omitted.

Various fixation devices, including interference screw, cross-pin, tab-loop anchor, screw-and-washer fixation devices, may be packaged with the replacement ligament, thus providing the surgeon with a kit for replacing a damaged ligament.

Synthetic materials may also be used in the practice of this invention. The graft material and/or bone plugs may be made of synthetic materials, and the replacement ligament may be constructed using a similar variety of techniques. The replacement ligament of this embodiment is then sterilized, and it is packaged according to the requirements of the materials used. As with allograft or xenograft replacement ligaments, the synthetic replacement ligaments may be packaged with or without graft fixation devices.

The replacement ligaments of this invention may be provided in a variety of lengths, with or without a variety of sizes of bone plugs. For ACL or PCL repair, lengths of 60 to 150 mm, preferably 90 to 100mm may be used, with bone plugs preferably having a diameter of 6 to 10 mm. However, certain PCL techniques may require longer grafts, and individual patients or alternative fixation techniques may require grafts of larger or smaller sizes. Also, grafts for use in the replacement of other ligaments or other soft tissue may be larger or smaller. Thus, while sizes are disclosed, it will be understood that these sizes are merely typical of sizes needed for repair of the cruciate ligaments. Other sized grafts are within the scope of this invention.

As an example of use, a replacement ligament 10 of this invention may be used to repair an anterior cruciate ligament. Fig. 15 illustrates the graft of the present invention after it has been inserted into the patient. The surgical site and the tibial tunnel 72 and femoral tunnel 70 are drilled as known in the art, see, for example, U.S. Patent No. 5,671,695, preferably while replacement ligament graft 10 is reconstituting or thawing. After tibial and femoral tunnels 72 and 70, respectively, have been drilled, a slotted guide pin (not shown) may then be passed through tibial tunnel 72 and into femoral tunnel 70. The knee is placed in hyperflexion and the guide pin can be passed out the anterolateral femoral cortex, where it then exits the skin.

Distal end long strand sutures 29 of the replacement ligament 10 are looped through the slotted guide pin eyelet (not shown) and pulled through the joint, exiting at the anterolateral femur. A pin puller, for example the DePuy Pin Puller (Cat. No. 2972-96-000), may be helpful in pulling the sutures through the joint. The graft is gently pulled into the tibial tunnel 72. A guidewire is then passed through a stab wound made at the level of the tibial plateau and just medial to the patellar tendon and into the femoral tunnel 70 while maintaining a position along the femoral wall. At this point the knee is flexed an additional 15-20 degrees beyond the flexion angle used to drill the femoral tunnel 70 to accommodate the guidewire position. A fixation device 66, such as the DePuy Phantom SofThread Screw, can be used to fix replacement ligament 10 into femoral tunnel 70.

After the guidewire has engaged the proximal aspect of the femoral tunnel 70, it is tapped into femur 80 with a mallet, for example the DePuy Mallet (Cat. No. 2972-23-000), to avoid rotation of the guidewire and the fixation device. A second guidewire is then passed through the tibial tunnel 72 anterior to graft 10. It may be useful to pull graft 10 back out of the tibial tunnel 72 to introduce the tibial wire.

After both wires are in position, the graft is then pulled up the tibial tunnel 72 and into the femoral tunnel 70. The proximal long strand sutures 30 are used to determine complete seating of graft 10 in femoral tunnel 70. Once the graft is tensioned both proximally and distally, the fixation device 66 is inserted through an anteromedial puncture wound.

The fixation device 66 is then advanced until the entire head is within femoral tunnel 70 and preferably countersunk beneath an edge of the femoral tunnel 70. The knee is then placed at approximately 20-30 degrees of flexion. Graft 10 is loaded to 9 kg (20 lbs.) 20 times by pulling on the long strand sutures 30 exiting the tibial tunnel 72. Using approximately 6.8 kg (15 lbs.) of preload, a tibial fixation device, for example tibial screw 67, is inserted along the anterior graft surface and advanced with driver 78 until it is completely within the tibial tunnel 72.

The knee is checked for mobility and stability and when acceptable, the proximal sutures 30 are pulled through the skin and removed. Sutures and any excess graft material flush with the tibial tunnel 72 should be transected. However, care should be taken to not cut the sutures that hold the graft together. The wounds are then closed in a standard fashion.

## Claims

1. A replacement package for the repair of a damaged ligament comprising a graft comprising a graft material (12) having a proximal end (45) and a distal end (46), in which the graft is provided without bone plugs and is preserved and provided in sterile packaging, **characterized in that** a first set of sutures (38) is attached to the proximal end (45), and a second set of sutures (38) is attached to the distal end (46).

2. A package as claimed in claim 1, in which the graft material (12) is selected from the group consisting of allograft, xenograft, and synthetic material.

3. A package as claimed in claim 2, in which the graft material (12) is allograft and selected from the group consisting of patellar tendon, semitendinosis tendon, gracilis tendon, quadriceps tendon, Achilles' tendons, flexor tendons, extensor tendons, or fascia lata.

4. A package as claimed in claim 1, in which the graft material (12) comprises a bundle of strands (14, 16), the first set of sutures (38) securing the proximal end (45) together, and the second set of sutures (38) securing the distal end together (46).

5. A package as claimed in claim 1, in which the first and second set of sutures comprise long strand sutures for aiding in subsequent placement of the graft into a patient.

6. A package as claimed in claim 1, in which the first and second set of sutures(38)each comprise whip stitch (39) sutures, bundle sutures, and long strand sutures.

7. A method of preparing a convenient replacement graft package for use in repairing a damaged ligament, the method comprising the steps of
removing extraneous tissue from a harvested piece of graft material;
washing the harvested piece of graft material;
cutting and shaping the piece of graft material to proper size, defining a proximal end and a distal end; **characterized in** further comprising the steps of
assembling the replacement graft without bone plugs by attaching a first set of sutures to the proximal end and attaching a second set of sutures to the distal end;
preserving the replacement graft; and
packaging the replacement graft in a sterile container.

8. A method as claimed in claim 7, in which the cutting and shaping step includes looping the piece of graft material back upon itself.

9. A method as claimed in claim 7, in which the washing step includes using a solution comprising mixed detergent solutions, isopropyl alcohol, and antibiotic solution.

10. A method as claimed in claim 9, in which the antibiotic solution comprises at least one of the group consisting of antibiotics, antiviral agents, hydrogen peroxide, permeation enhancers, organic acids, dilute solutions of strong acids, and a mixture of bacitracin and polymyxin.

11. A method as claimed in claim 7, in which the packaging step includes placing the graft in a bottle, the preserving step includes lyophilization and the graft is placed in the bottle prior to lyophilization.

12. A method as claimed in claim 11, in which the packaging step includes stoppering the bottle under vacuum following lyophilization.

13. A method as claimed in claim 7, in which the preserving step comprises freezing and the packaging step comprises placing the graft in a peelable soft package, removing air by suction, heat-sealing the package, and freezing the package.

14. A method as claimed in claim 13, in which the packaging step includes placing the peelable soft package into a second larger package.

15. A method as claimed in claim 13, in which the package is irradiated prior to freezing.

16. A method as claimed in claim 7, which includes the step of placing the graft under tension prior to the preserving step.

17. A kit for replacing a damaged ligament in a patient comprising a sterile packaged prepared replacement ligament according to any one of claims 1 to 6, and a graft fixation device.

18. A kit as claimed in claim 18, in which the graft fixation device is selected from the group consisting of interference screws, cross pins, tab-loop anchors, and screws and washers.

## Patentansprüche

1. Austauschpackung für die Reparatur eines beschädigten Bands, umfassend ein Transplantat, das ein Transplantatmaterial (12) umfaßt, welches ein proximales Ende (45) und ein distales Ende (46) aufweist, wobei das Transplantat ohne Knochendübel bereitgestellt ist und in einer sterilen Verpackung konserviert und bereitgestellt ist,
**dadurch gekennzeichnet, daß**
ein erster Satz von Fäden (38) am proximalen Ende (45) angebracht ist, und ein zweiter Satz von Fäden (38) am distalen Ende (46) angebracht ist.

2. Packung nach Anspruch 1, wobei das Transplantatmaterial (12) aus der Gruppe gewählt wird, bestehend aus artgleichen Transplantaten, artfremden Transplantaten und künstlichen Materialien.

3. Packung nach Anspruch 2, wobei das Transplantatmaterial (12) ein artgleiches Transplantat ist und aus der Gruppe gewählt wird, die aus der Patellarsehne, der Semitendinosussehne, der Gracilissehne, der Quadricepssehne, Achillessehnen, Flexorsehnen, Extensorsehnen oder der breiten Fascie des Oberschenkels besteht.

4. Packung nach Anspruch 1, wobei das Transplantatmaterial (12) ein Bündel von Strängen (14, 16) umfaßt, wobei der erste Satz von Fäden (38) das proximale Ende (45) zusammenspannt und der zweite Satz von Fäden (38) das distale Ende (46) zusammenspannt.

5. Packung nach Anspruch 1, wobei der erste und der zweite Satz von Fäden lange Strangfäden umfaßt, um die anschließende Anordnung des Transplantats in einem Patienten zu unterstützen.

6. Packung nach Anspruch 1, wobei der erste und der zweite Satz (38) jeweils Peitschennahtfäden (39), Bündelfäden und lange Strangfäden umfaßt.

7. Verfahren zum Anfertigen einer praktischen Austauschtransplantatpackung zur Verwendung bei der Reparatur eines beschädigten Bands, wobei das Verfahren die folgenden Schritte umfaßt:
Entfernen von Fremdgewebe von einem eingesammelten Stück eines Transplantatmaterials;
Waschen des eingesammelten Stücks des Transplantatmaterials;
Schneiden und Formen des Stücks des Transplantatmaterials in eine richtige Größe, wodurch ein proximales Ende und ein distales Ende definiert wird;
**dadurch gekennzeichnet, daß** das Verfahren ferner die folgenden Schritte umfaßt:
Zusammensetzen des Austauschtransplantats ohne Knochendübel durch Anbringen eines ersten Satzes von Fäden am proximalen Ende und Anbringen eines zweiten Satzes von Fäden am distalen Ende;
Konservieren des Austauschtransplantats; und
Verpacken des Austauschtransplantats in einem sterilen Behälter.

8. Verfahren nach Anspruch 7, wobei der Schritt des Schneidens und Formens das Zurückschlingen des Stücks des Transplantatmaterials auf sich selbst beinhaltet.

9. Verfahren nach Anspruch 7, wobei der Schritt des Waschens das Verwenden einer Lösung beinhaltet, die gemischte Waschmittellösungen, Isopropylalkohol und eine antibiotische Lösung umfaßt.

10. Verfahren nach Anspruch 9, wobei die antibiotische Lösung zumindest eines aus der Gruppe umfaßt, die aus Antibiotika, Antivirusmitteln, Wasserstoffperoxid, Durchdringungsverstärkern, organischen Säuren, verdünnten Lösungen von starken Säuren und einem Gemisch aus Bacitracin und Polymyxin besteht.

11. Verfahren nach Anspruch 7, wobei der Schritt des Verpackens das Anordnen des Transplantats in einer Flasche beinhaltet, und der Schritt des Konservierens eine Lyophilisierung beinhaltet, und das Transplantat vor der Lyophilisierung in der Flasche angeordnet wird.

12. Verfahren nach Anspruch 11, wobei der Schritt des Verpackens das Verstöpseln der Flasche unter Vakuum im Anschluß an die Lyophilisierung beinhaltet.

13. Verfahren nach Anspruch 7, wobei der Schritt des Konservierens Gefrieren umfaßt, und der Schritt des Verpackens das Anordnen des Transplantats in einer abziehbaren weichen Packung, das Entfernen der Luft durch Absaugen, das Heißverschweißen der Packung und das Gefrieren der Packung umfaßt.

14. Verfahren nach Anspruch 13, wobei der Schritt des Verpackens das Anordnen der abziehbaren weichen Packung in einer zweiten größeren Packung beinhaltet.

15. Verfahren nach Anspruch 13, wobei die Packung vor dem Gefrieren bestrahlt wird.

16. Verfahren nach Anspruch 7, das vor dem Schritt des Konservierens den Schritt des Anordnens des Transplantats unter Spannung beinhaltet.

17. Ausrüstungssatz zum Austauschen eines beschädigten Bands in einem Patienten, umfassend ein steril verpackt angefertigtes Austauschband nach einem der Ansprüche 1 bis 6 und eine Transplantatbefestigungsvorrichtung.

18. Ausrüstungssatz nach Anspruch 17, wobei die Transplantatbefestigungsvorrichtung aus der Gruppe gewählt wird, die aus Interferenzschrauben, Kreuzstiften, Laschen-Ösen-Verankerungen und Kombischrauben besteht.

## Revendications

1. Ensemble de mesures de remplacement pour la remise en état d'un ligament endommagé comprenant un greffon comprenant un matériau de greffe (12) ayant une extrémité proximale (45) et une extrémité distale (46), dans lequel le greffon est prévu sans chevilles osseuses et est préservé et fourni dans un emballage stérile, **caractérisé en ce qu'**une première série de points de suture (38) est attachée à l'extrémité proximale (45) et qu'une seconde série de points de suture (38) est attachée à l'extrémité distale (46).

2. Ensemble de mesures comme revendiqué dans la revendication 1, dans lequel le matériau de greffe (12) est choisi parmi le groupe se composant de l'allogreffe, du xénogreffe et du matériau synthétique.

3. Ensemble de mesures comme revendiqué dans la revendication 2, dans lequel le matériau de greffe (12) est l'allogreffe et est choisi parmi le groupe se composant du tendon rotulien, du tendon semi-tendineux, du tendon du muscle gracile, du tendon du quadriceps, du tendon d'Achille, des tendons fléchisseurs, des tendons extenseurs ou du fascia lata.

4. Ensemble de mesures comme revendiqué dans la revendication 1, dans lequel le matériau de greffe (12) comprend un paquet de fibres (14, 16), la première série de points de suture (38) attachant ensemble l'extrémité proximale (45) et la seconde série de points de suture (38) attachant ensemble l'extrémité distale (46).

5. Ensemble de mesures comme revendiqué dans la revendication 1, dans lequel la première série et la seconde série de points de suture comprennent des points de suture à longue fibre pour aider au placement ultérieur du greffon dans un patient.

6. Ensemble de mesures comme revendiqué dans la revendication 1, dans lequel la première série et la seconde série de points de suture (38) comprennent chacune des points de suture avec un point en surjet (39), des points de suture en paquet et des points de suture à longue fibre.

7. Procédé consistant à préparer un ensemble de mesures pratiques pour une greffe de remplacement pour une utilisation dans la remise en état d'un ligament endommagé, le procédé comprenant les étapes consistant à :
enlever le tissu étranger d'une pièce du matériau de greffe récoltée;
laver la pièce du matériau de greffe récoltée ;
couper et façonner la pièce du matériau de greffe à la bonne taille, en définissant une extrémité proximale et une extrémité distale ; **caractérisé** davantage dans le fait de comprendre les étapes consistant à :
assembler le greffon de remplacement sans prises osseuses en attachant une première série de points de suture à l'extrémité proximale et en attachant une seconde série de points de suture à l'extrémité distale ;
conserver le greffon de remplacement ; et
emballer le greffon de remplacement dans un conteneur stérile.

8. Procédé comme revendiqué dans la revendication 7, dans lequel l'étape consistant à couper et à façonner inclut le bouclage de la pièce du matériau de greffe sur elle-même.

9. Procédé comme revendiqué dans la revendication 7, dans lequel l'étape de lavage inclut l'utilisation d'une solution comprenant des solutions détergentes, un alcool isopropylique et une solution antibiotique mélangés.

10. Procédé comme revendiqué dans la revendication 9, dans lequel la solution antibiotique comprend au moins l'un du groupe se composant des antibiotiques, des agents antiviraux, du peroxyde d'hydrogène, d'exhausteurs de pénétration, d'acides organiques, de solutions diluées de puissants acides et d'un mélange de bacitracine et de polymyxine.

11. Procédé comme revendiqué dans la revendication 7, dans lequel l'étape d'emballage inclut le placement du greffon dans un flacon, l'étape de conservation inclut la lyophilisation et le greffon est placé dans le flacon avant la lyophilisation.

12. Procédé comme revendiqué dans la revendication 11, dans lequel l'étape d'emballage inclut le fait de fermer le flacon sous vide après la lyophilisation.

13. Procédé comme revendiqué dans la revendication 7, dans lequel l'étape de conservation comprend la congélation et l'étape d'emballage comprend le placement du greffon dans un emballage souple qui peut être enlevé, l'enlèvement de l'air par aspiration, le thermosoudage de l'emballage et la congélation de l'emballage.

14. Procédé comme revendiqué dans la revendication 13, dans lequel l'étape d'emballage inclut le placement de l'emballage souple, qui peut être enlevé, dans un second emballage plus grand.

15. Procédé comme revendiqué dans la revendication 13, dans lequel l'emballage est irradié avant la congélation.

16. Procédé comme revendiqué dans la revendication 7, qui inclut l'étape consistant à placer le greffon sous tension avant l'étape de conservation.

17. Kit pour remplacer un ligament endommagé dans un patient comprenant un ligament de remplacement préparé, conditionné, stérile selon l'une des revendications 1 à 6 et un dispositif de fixation de greffon.

18. Kit comme revendiqué dans la revendication 18, dans lequel le dispositif de fixation de greffon est choisi parmi le groupe se composant de vis d'interférence, de croisillons, de points d'ancrage de type lacet - boucle, et de vis et de rondelles.
